# EUROPÄISCHE PATENTANMELDUNG

(11) **EP 4 846 985 A1**
(43) Veröffentlichungstag der Anmeldung: **02.12.2026**
(21) Anmeldenummer: 25179374.1
(22) Anmeldetag: 28.05.2025
(51) Int. Cl.: A61F 5/08

(54) **EXTERNER NASENDILATATOR MIT WIRKSTOFFSCHICHT**

(71) Anmelder: OG Service GmbH, 85296 Rohrbach (DE)
(72) Erfinder: KOC, Ahmet Taha, 85356 Freising (DE)
(74) Vertreter: Betten & Resch

(57) **Zusammenfassung**

Es wird eine externer Nasendilatator beschrieben, der eine Zugschicht und eine Wirkstoffschicht umfasst, wobei die Zugschicht eine Zugkraft auf die Nasenflügel ausübt und die Wirkstoffschicht einen Wirkstoff transdermal an die Nasenhaut abgibt.

## Beschreibung

### TECHNISCHES GEBIET

Die vorliegende Offenbarung betrifft einen externen Nasendilatator zum Weiten von Nasenflügeln. Insbesondere betrifft die vorliegende Offenbarung auf einen externen Nasendilatator mit Wirkstoffschicht zur transdermalen Abgabe eines Wirkstoffs an eine Nasenhaut.

### HINTERGRUND

Nasendilatatoren sind Vorrichtungen, die dazu dienen, Nasenflügel nach außen zu ziehen und so die Nasenflügel mechanisch zu weiten, um den Strömungswiderstand in den Nasenflügeln zu verringern, bzw. den nasalen Luftstrom zu verbessern. Diese Vorrichtungen unterstützen insbesondere bei hoher Belastung, z.B. beim Sport, beim Schlafen oder bei anatomisch bedingter Nasenatmungseinschränkung. Auf dem Gebiet der Nasendilatatoren sind externe und interne Vorrichtungen bekannt. Externe Nasendilatatoren werden üblicherweise auf die Nasenhaut aufgeklebt, wobei durch elastische Zugstreifen im Nasendilatator eine Zugkraft auf die Nasenflügel ausgeübt wird, die die Nasenflügel weitet. Ferner existieren interne Dilatatoren, die durch Spreizwirkung die Nasenflügel weiten und zumindest teilweise in die Nase eingeführt werden müssen.

Wirkstoffpflaster, z.B. ein Nikotinpflaster, insbesondere transdermale therapeutische Systeme (TTS), sind Vorrichtungen zur kontrollierten Abgabe eines Wirkstoffs über die Haut in den Blutkreislauf. Ein etabliertes Anwendungsgebiet ist die Raucherentwöhnung mittels Nikotinpflastern, die an den Körper über einen gewissen Zeitraum Nikotin abgeben, um Entzugssymptome eines Rauchers zu lindern und den Suchtdruck zu reduzieren. Derartige Pflastersysteme ermöglichen eine diskrete, nicht-invasive Anwendung und bieten konstante Wirkstoffzufuhr. Neben Nikotin kommen transdermale Systeme auch bei anderen Indikationen, wie z.B. in der Schmerztherapie zur Verwendung.

Raucher leiden häufig unter Atemproblemen, die durch die schädlichen Auswirkungen des Tabakkonsums auf die Atemwege verursacht werden. Langfristiges Rauchen kann zu einer chronischen Reizung der Schleimhäute, einer Verengung der Atemwege und einer verminderten Lungenfunktion führen und Symptome wie Kurzatmigkeit, Husten und einer erhöhten Anfälligkeit für Atemwegserkrankungen verursachen.

Ein Problem ist, dass die Folgeschäden des langfristigen Rauchens meist unabhängig und entkoppelt von der Suchttherapie, z.B. über Nikotinpflaster, betrachtet werden. Während Nikotinpflaster und andere Entwöhnungsmaßnahmen zur Bekämpfung der Nikotinabhängigkeit angewandt werden, werden Symptome wie Verengung der Atemwege und Atemprobleme als separate Herausforderung häufig nicht berücksichtigt. Dies führt zu einer unvollständigen Therapie der Langzeitfolgen des Rauchens, da die körperliche Erholung der Atemwege nicht gleichzeitig mit der Suchtbekämpfung angegangen wird.

Ein weiteres Problem besteht darin, dass selbst wenn die Suchtprobleme und Atemprobleme gleichzeitig behandelt werden, in der Regel zwei separate Maßnahmen zum Einsatz kommen. So werden zur Suchtbekämpfung z.B. transdermale Wirkstoffpflaster wie etwa Nikotinpflaster verwendet, während Atemprobleme z.B. mit Mitteln wie Atemtherapie, Inhalatoren oder Dilatatoren behandelt werden. Diese Trennung der Behandlungsmaßnahmen bedeutet, dass Betroffene mehrere Maßnahmen gleichzeitig ergreifen müssen, was den Komfort beeinträchtigt und die Anwendung erschwert.

Es ist daher ein der vorliegenden Erfindung zugrunde liegendes technisches Problem, eine Vorrichtung zur ganzheitlichen Behandlung sowohl von Suchtproblemen als auch von Atemproblemen, bereitzustellen, die die Nachteile bekannter Systeme überwindet.

### ZUSAMMENFASSUNG

Im Folgenden werden einige Ausführungsformen der Offenbarung zusammengefasst, um ein grundlegendes Verständnis dieser Ausführungsformen und ihrer Vorteile zu vermitteln. Weitere Ausführungsformen und technische Details sind in der nachstehenden detaillierten Beschreibung ausgeführt.

Ein externer Nasendilatator umfasst eine Zugschicht, konfiguriert zum Ausüben einer Zugkraft auf Nasenflügel, sodass die Nasenflügel zu geweiteten Nasenflügeln geweitet werden, wenn der externe Nasendilatator auf einer Nase getragen wird. Der externe Nasendilatator umfasst ferner eine Wirkstoffschicht, konfiguriert zum transdermalen Abgeben eines Wirkstoffs an eine Nasenhaut, wobei die Zugschicht oberhalb der Wirkstoffschicht angeordnet ist. Somit vereint der externe Nasendilatator zwei Funktionen, nämlich die Zugkraft der Zugschicht und die daraus resultierende Weitung der verengten Nasenflügel zu geweiteten Nasenflügeln, sowie die transdermale Abgabe eines Wirkstoffs über die Wirkstoffschicht.

In einigen Ausführungsformen besteht die Zugschicht aus einem elastisch verformbaren Material. Die Zugschicht ist derart vorgespannt, dass die auf die Nasenflügel ausgeübte Zugkraft die Nasenflügel quer zum Nasenrücken der Nase zu geweiteten Nasenflügeln weitet. Die zum Nasenrücken der Nase quere Anordnung des externen Nasendilatators bewirkt, dass die Zugschicht die verengten Nasenflügel durch eine laterale Zugkraft weitet. Durch diese Anordnung ist die Kraftverteilung ferner gleichmäßig über die Fläche der Nasenhaut, auf der der externe Nasedilatator fixiert ist, verteilt, was den Tragekomfort erhöht und die Anpassung an unterschiedliche Nasenformen erleichtert.

In einigen Ausführungsformen ist der Wirkstoff einer aus Nikotin, Tetrahydrocannabinol, THC, oder Cannabidiol, CBD. Der externe Nasendilatator ermöglicht über die Wirkstoffschicht eine kontinuierliche und kontrollierte Wirkstoffabgabe auf eine nicht-invasive und anwenderfreundliche Weise. Dadurch wird eine stabile Wirkstoffkonzentration im Körper erreicht, die Entzugssysteme reduziert und das Verlangen nach Substanzen wie Nikotin, THC oder CBC reduziert. Die kontrollierte Wirkstoffabgabe vermindert ferner Rückfallrisiken, da keine schnellen Wirkungsspitzen auftreten, sondern ein konstantes Wirkstoffniveau gehalten wird. Der Entzugsprozess wird durch die Wirkstoffschicht des externen Nasendilatators auch insofern unterstützt, als dass die Wirkstoffdosierung individuell definiert werden kann und somit über einen Zeitraum kontinuierlich reduziert werden kann.

In einigen Ausführungsformen enthält die Wirkstoffschicht ferner Aromastoffe und/oder feuchtigkeitsspendende Substanzen und/oder oder sensorische Elemente. Die Integration einer oder mehrere dieser zusätzlichen Stoffe in die Wirkstoffschicht des externen Nasendilatators kann das subjektive Wohlbefinden während des Entzugs fördern und dadurch die Applikationsbereitschaft erhöhen.

In einer ersten Ausführungsform ist die Wirkstoffschicht als selbstklebende Matrix-Wirkstoffschicht ausgebildet, konfiguriert zum Fixieren des externen Nasendilatators auf der Nasenhaut und zum transdermalen Abgeben des Wirkstoffs an die Nasenhaut über ein Matrixsystem. Bei der selbstklebenden Matrix-Wirkstoffschicht ist der Wirkstoff gleichmäßig in der Schicht eingelagert und durch die direkte Haftung auf der Nasenhaut erfolgt eine gleichmäßige und kontrollierte transdermale Abgabe an die Nasenhaut. Im Falle einer selbstklebenden Matrix-Wirkstoffschicht dient die Matrix sowohl als Wirkstoffspeicher, als auch zur Fixierung des externen Nasendilatators auf der Nase.

In einer zweiten Ausführungsform ist die Wirkstoffschicht als Matrix-Wirkstoffschicht ausgebildet, konfiguriert zum transdermalen Abgeben des Wirkstoffs an die Nasenhaut über ein Matrixsystem. Ist die Wirkstoffschicht als Matrix-Wirkstoffschicht ausgebildet, so umfasst der externe Nasendilatator eine unter der Wirkstoffschicht liegende Klebeschicht zum Fixieren des externen Nasendilatators auf der Nase. Die Matrix-Wirkstoffschicht dient als Wirkstoffträger, in der der Wirkstoff gleichmäßig verteilt ist. Nach Applikation des externen Nasendilatators auf der Nasenhaut wird der Wirkstoff über ein Konzentrationsgefälle freigesetzt und diffundiert durch die Nasenhaut in den Blutkreislauf. Die Matrix-Wirkstoffschicht gibt den Wirkstoff nach Applikation auf der Nase so lange kontinuierlich ab, wie ein ausreichendes Konzentrationsgefälle zwischen dem Wirkstoff in der Matrix-Wirkstoffschicht und der Nasenhaut vorliegt.

In einer dritten Ausführungsform umfasst die Wirkstoffschicht eine Reservoirschicht und eine Membranschicht, wobei die Membranschicht unterhalb der Reservoirschicht angeordnet ist, und wobei die Reservoirschicht, zum kontrollierten transdermalen Abgeben des Wirkstoffs an die Nasenhaut über die Membranschicht konfiguriert ist. Der Wirkstoff ist in der Reservoirschicht in gelöster oder dispergierter Form enthalten. Die Membranschicht dient als kontrollierende Barriere zwischen der Reservoirschicht und der Nasenhaut, durch die der Wirkstoff mit einer bestimmten Rate abgegeben wird. Die Abgabe erfolgt über Diffusion durch die Nasenhaut über ein Konzentrationsgefälle zwischen dem Wirkstoff in der Reservoirschicht und der Nasenhaut, wobei die Membranschicht sicherstellt, dass die Abgabegeschwindigkeit unabhängig von der verbleibenden Wirkstoffmenge in der Reservoirschicht konstant bleibt, wodurch eine gleichbleibende Dosierung des Wirkstoffs über den Zeitraum der Applikation gewährleistet wird.

In einer vierten Ausführungsform ist die Wirkstoffschicht als mikroverkapselter Film ausgebildet, wobei der mikroverkapselte Film eine Mehrzahl verkapselter Wirkstoffdepots umfasst, und wobei jedes der Mehrzahl verkapselter Wirkstoffdepots zum kontrollierten transdermalen Abgeben des Wirkstoffs an die Nasenhaut konfiguriert ist. Ist die Wirkstoffschicht als mikroverkapselter Film ausgebildet, umfasst der mikroverkapselte Film eine Mehrzahl von Wirkstoffdepots. Jedes Wirkstoffdepot der Mehrzahl von Wirkstoffdepots enthält den Wirkstoff in verkapselter Form, sodass eine Abgabe des Wirkstoffs an die Nasenhaut kontrolliert verzögert wird. Die Mehrzahl der Wirkstoffdepots ist gleichmäßig über die Wirkstoffschicht verteilt, sodass eine definierte gleichmäßige Abgabe über die gesamte Applikationsfläche ermöglicht wird.

In einigen Ausführungsformen umfasst der externe Nasendilatator eine Klebeschicht, konfiguriert zum Fixieren des externen Nasendilatators auf der Nasenhaut, wobei die Klebeschicht unterhalb der Wirkstoffschicht angeordnet ist. Die Klebeschicht ist derart ausgestaltet, dass sie eine dauerhafte, aber reversible Haftung an der Nasenhaut ermöglicht, insbesondere im Bereich von Nasenflügeln der sich durch Bewegung, Feuchtigkeit und hohe Sensibilität auszeichnet. Ferner weist die Klebeschicht eine ausreichende Haftfähigkeit auf, die eine dauerhafte Fixierung des externen Nasendilatators an der Nase ermöglicht, auch wenn durch die Zugschicht die Zugkraft auf die Nasenflügel ausgeübt wird.

In einigen Ausführungsformen umfasst der externe Nasendilatator eine Trägerschicht, die zum Schützen vor äußeren Einwirkungen konfiguriert ist, und wobei die Trägerschicht oberhalb der der Zugschicht angeordnet ist. Die Trägerschicht ist als mechanisch flexible Oberschicht des externen Nasendilatators angeordnet und ist derart konfiguriert, dass sie die darunterliegenden Schichten vor äußeren Einflüssen wie Feuchtigkeit, Schmutz oder mechanischer Belastung schützt.

In einigen Ausführungsformen umfasst der externe Nasendilatator eine entfernbare Schutzschicht, die zum Schützen vor äußeren Einwirkungen vor Applikation des externen Nasendilatators auf der Nasenhaut konfiguriert ist, wobei die entfernbare Schutzschicht als unterste Schicht des externen Nasendilatators angeordnet ist. Die entfernbare Schutzschicht ist als unterste Schicht angeordnet und derart konfiguriert, dass sie die darüberliegenden Schichten vor äußeren Einwirkungen wie Feuchtigkeit, Schmutz oder mechanischer Belastung schützt. Die entfernbare Schutzschicht kann vor der Applikation des externen Nasendilatators von dem externen Nasendilatator, zum Beispiel durch Abziehen, entfernt werden, sodass die darüberliegende Schicht des externen Nasendilatators zur untersten Schicht wird, welche auf der Nasenhaut angebracht werden kann.

Die vorhergehende allgemeine Beschreibung und die nachfolgende detaillierte Beschreibung dienen lediglich der Veranschaulichung und Erläuterung und schränken die beanspruchte Erfindung nicht ein.

### LISTE DER FIGUREN

FIG. 1a zeigt eine Nase mit verengten Nasenflügeln.
FIG. 1b zeigt eine Nase nach Applikation eines externen Nasendilatators mit geweiteten Nasenflügeln.
FIG. 2 zeigt eine Applikation eines externen Nasendilatators auf einer Nase.
FIG. 3 zeigt eine schematische Darstellung des Schichtaufbaus eines externen Nasendilatators mit einer Wirkstoffschicht.
FIG. 4a zeigt eine schematische Darstellung des Schichtaufbaus eines externen Nasendilatators gemäß einem Beispiel mit einer selbstklebenden Matrix-Wirkstoffschicht.
FIG. 4b zeigt eine schematische Darstellung des Schichtaufbaus eines externen Nasendilatators gemäß einem Beispiel mit einer Matrix-Wirkstoffschicht.
FIG. 4c zeigt eine schematische Darstellung des Schichtaufbaus eines externen Nasendilatators gemäß einem Beispiel mit einer Reservoirschicht als Wirkstoffschicht.
FIG. 4d zeigt eine schematische Darstellung des Schichtaufbaus eines externen Nasendilatators gemäß einem Beispiel mit einem mikroverkapselten Film als Wirkstoffschicht.

### DETAILLIERTE BESCHREIBUNG

Im Folgenden wir ein externer Nasendilatator beschrieben, der eine Zugschicht und eine Wirkstoffschicht umfasst. Die Zugschicht übt eine Zugkraft auf die Nasenflügel aus. Die Wirkstoffschicht gibt einen Wirkstoff transdermal an die Nasenhaut ab. Eine derartige Vorrichtung ermöglicht die kombinierte und gleichzeitige Behandlung von Sucht- und Atemproblemen und löst die zuvor geschilderten Probleme aus dem Stand der Technik zumindest teilweise.

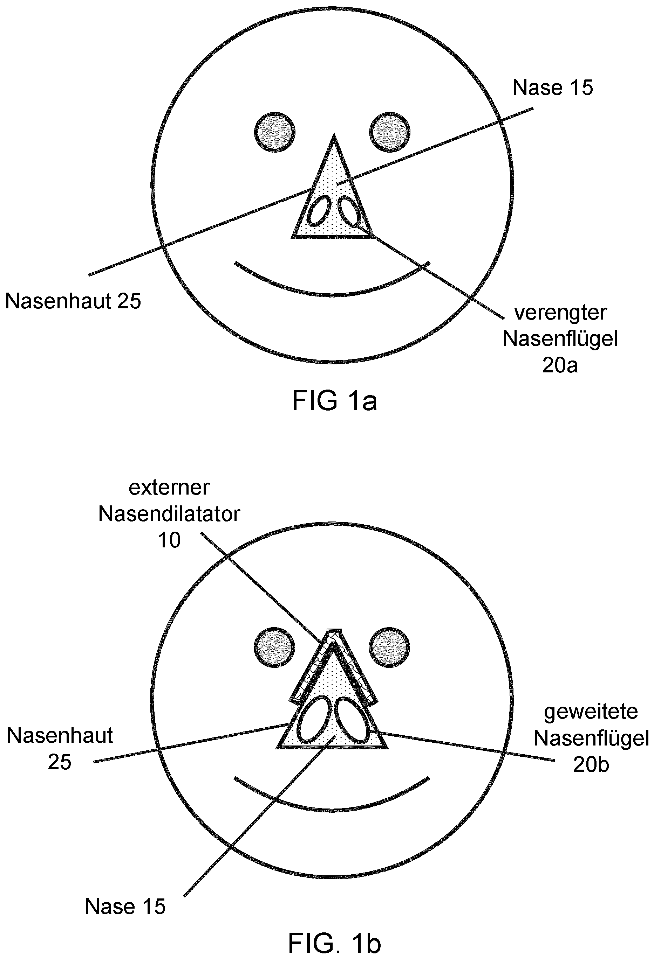

FIG. 1a zeigt eine Nase 15 mit einer Nasenhaut 25 und Nasenflügeln 20a. Die verengten Nasenflügel 20a können, z.B. aufgrund von Rauchen, verengt sein, beispielsweise weil Teile der Nase 15 verstopft sind oder Nasenschleimhäute entzündet sind.

FIG. 1b zeigt eine Nase 15 nach Applikation eines externen Nasendilatators 10. Der an der Nasenhaut 25 angebrachte externe Nasendilatator 10 bewirkt eine Weitung der verengten Nasenflügel 20a zu geweiteten Nasenflügeln 20b, indem eine Zugkraft auf die verengten Nasenflügel 20a ausgeübt wird. Durch die mechanische Weitung der verengten Nasenflügel 20a zu geweiteten Nasenflügeln 20b wird der Strömungswiderstand in den Nasenflügeln verringert und der nasale Luftstrom verbessert. Dies wirkt potenziellen Atemproblemen entgegen, indem die Atemwege geweitet werden.

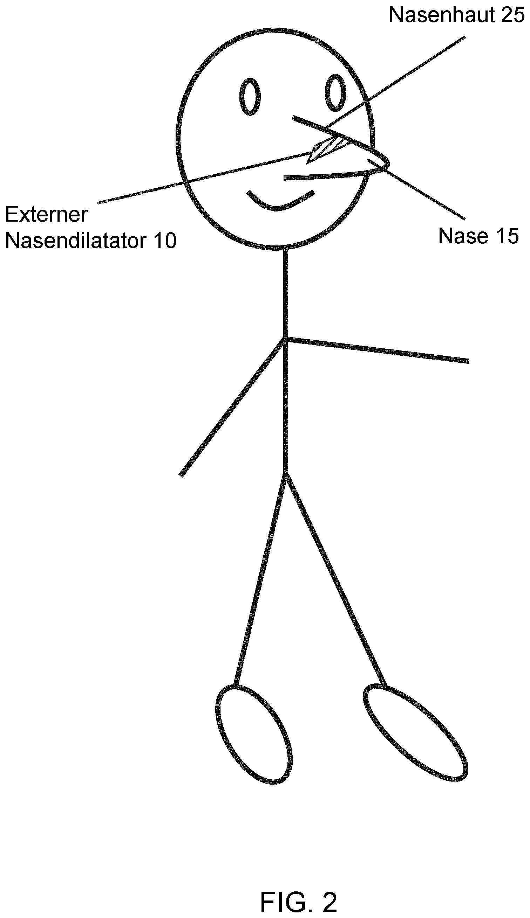

FIG. 2 zeigt eine Applikation eines externen Nasendilatators 10 auf einer Nase 15. Der externe Nasendilatator 10 ist auf beiden Seiten der Nase 15 quer zum Nasenrücken der Nase 15 fixiert, sodass die Zugkraft auf die jeweiligen verengten Nasenflügel 20a auf beiden Seiten des Nasenrückens der Nase 15 ausgeübt wird und so zu geweiteten Nasenflügeln 20b geweitet werden.

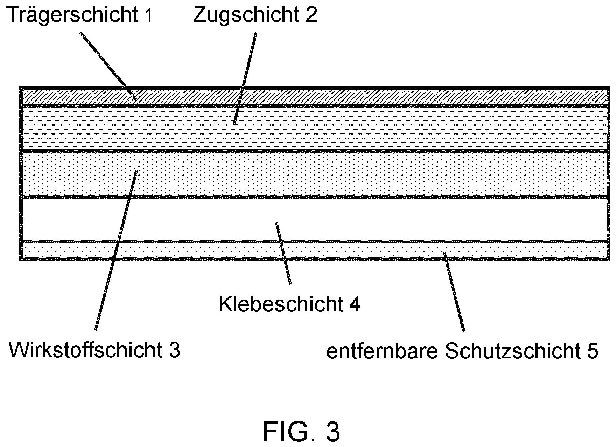

FIG. 3 zeigt eine schematische Darstellung des Schichtaufbaus eines externen Nasendilatators 10 mit einer Wirkstoffschicht 3. Der externe Nasendilatator 10 weist einen Schichtaufbau auf, wobei die unterste Schicht in Kontakt mit der Nasenhaut 25 steht. Der externe Nasendilatator 10, umfasst eine Zugschicht 2, konfiguriert zum Ausüben einer Zugkraft auf verengte Nasenflügel 20a, sodass die verengten Nasenflügel 20a zu geweiteten Nasenflügeln 20b geweitet werden, wenn der externe Nasendilatator 10 auf der Nase 15 getragen wird. Ferner umfasst der externe Nasendilatator 10 eine Wirkstoffschicht 3, konfiguriert zum transdermalen Abgeben eines Wirkstoffs an die Nasenhaut 25, wobei die Wirkstoffschicht 3 unterhalb der Zugschicht 2 angeordnet ist. Somit vereint der externe Nasendilatator 10 zwei Funktionen, nämlich die Zugkraft der Zugschicht 2 und die daraus resultierende Weitung der verengten Nasenflügel 20a zu geweiteten Nasenflügeln 20b, sowie die transdermale Abgabe eines Wirkstoffs über die Wirkstoffschicht 3.

Die Zugschicht 2 kann aus einem elastisch verformbaren Material bestehen und derart vorgespannt sein, dass auf die verengten Nasenflügel 20a eine Zugkraft ausgeübt wird, die die verengten Nasenflügel 20a quer zum Nasenrücken der Nase 15 zu geweiteten Nasenflügeln 20b weitet. Die zum Nasenrücken der Nase 15 quere Anordnung des externen Nasendilatators 10 bewirkt, dass die Zugschicht 2 die verengten Nasenflügel 20a durch eine laterale Zugkraft weitet. Durch diese Anordnung ist die Kraftverteilung ferner gleichmäßig über die Fläche der Nasenhaut 25, auf der der externe Nasedilatator 10 fixiert ist, verteilt, was den Tragekomfort erhöht und die Anpassung an unterschiedliche Nasenformen erleichtert.

Die Wirkstoffschicht 3 enthält einen Wirkstoff der transdermal an die Nasenhaut 25 abgegeben wird. Der Wirkstoff kann einer aus Nikotin, Tetrahydrocannabinol, THC, oder Cannabidiol, CBD sein. Der externe Nasendilatator 10 ermöglicht über die Wirkstoffschicht 3 eine kontinuierliche und kontrollierte Wirkstoffabgabe auf eine nicht-invasive und anwenderfreundliche Weise. Dadurch wird eine stabile Wirkstoffkonzentration im Körper erreicht, die Entzugssysteme reduziert und das Verlangen nach Substanzen wie Nikotin, THC oder CBC reduziert. Die kontrollierte Wirkstoffabgabe vermindert ferner Rückfallrisiken, da keine schnellen Wirkungsspitzen auftreten, sondern ein konstantes Wirkstoffniveau gehalten wird. Der Entzugsprozess wird durch die Wirkstoffschicht 3 des externen Nasendilatators 10 auch insofern unterstützt, als dass die Wirkstoffdosierung individuell definiert werden kann und somit über einen Zeitraum kontinuierlich reduziert werden kann.

Zusätzlich zum Wirkstoff kann die Wirkstoffschicht 3 ferner zusätzliche Stoffe wie Aromastoffe, feuchtigkeitsspendende Substanzen und/oder sensorische Elemente (wie z.B. Menthol) enthalten. Die Integration einer oder mehrere dieser zusätzlichen Stoffe in die Wirkstoffschicht 3 des externen Nasendilatators 10 kann das subjektive Wohlbefinden während des Entzugs fördern und dadurch die Applikationsbereitschaft erhöhen.

Optional umfasst der externe Nasendilatator 10 zusätzlich zu der Zugschicht 2 und der Wirkstoffschicht 3, auch eine Trägerschicht 1, eine Klebeschicht 4 und/oder eine entfernbare Schutzschicht 5. Der jeweilige Schichtaufbau des externen Nasendilatators 10 ergibt sich aus der Anordnung der Schichten wie in FIG. 3 gezeigt. Umfasst der externe Nasendilatator 10 zum Beispiel neben der Zugschicht 2 und der Wirkstoffschicht 3 auch eine Trägerschicht 1, so ist diese Schicht oberhalb der Zugschicht 2 angeordnet. Umfasst der externe Nasendilatator 10 zusätzlich eine Klebeschicht 4, so ist diese unterhalb der Wirkstoffschicht 3 angeordnet. Umfasst der externe Nasendilatator 10 eine entfernbare Schutzschicht 5, so ist diese stets als unterste Schicht unter allen anderen Schichten des externen Nasendilatators 10 angeordnet.

Die optionale Trägerschicht 1 ist als mechanisch flexible Oberschicht des externen Nasendilatators 10 angeordnet und ist derart konfiguriert, dass sie die darunterliegenden Schichten vor äußeren Einflüssen wie Feuchtigkeit, Schmutz oder mechanischer Belastung schützt.

Die optionale Klebeschicht 4 ist unterhalb der Wirkstoffschicht 3 angeordnet und derart konfiguriert, dass sie ein Fixieren des externen Nasendilatators 10 durch Aufkleben auf der Nasenhaut 25 ermöglicht. Die Klebeschicht 4 ist vorzugsweise derart ausgestaltet, dass sie eine dauerhafte, aber reversible Haftung an der Nasenhaut 25 ermöglicht, insbesondere im Bereich von Nasenflügeln 20a, 20b der sich durch Bewegung, Feuchtigkeit und hohe Sensibilität auszeichnet. Die Klebeschicht 4 besteht vorzugsweise aus einem hautfreundlichen druckempfindlichen Klebstoff, der eine reizfreie Applikation und Entfernung des externen Nasendilatators 10 erlaubt. Ferner weist die Klebeschicht 4 eine ausreichende Haftfähigkeit auf, die eine dauerhafte Fixierung des externen Nasendilatators 10 an der Nase ermöglicht, auch wenn durch die Zugschicht 2 die Zugkraft auf die Nasenflügel ausgeübt wird.

Die optionale entfernbare Schutzschicht 5 ist als unterste Schicht angeordnet und derart konfiguriert, dass sie die darüberliegenden Schichten vor äußeren Einwirkungen wie Feuchtigkeit, Schmutz oder mechanischer Belastung schützt. Die entfernbare Schutzschicht 5 kann vor der Applikation des externen Nasendilatators 10 von dem externen Nasendilatator 10, zum Beispiel durch Abziehen, entfernt werden, sodass die darüberliegende Schicht des externen Nasendilatators zur untersten Schicht wird, welche auf der Nasenhaut 25 angebracht werden kann.

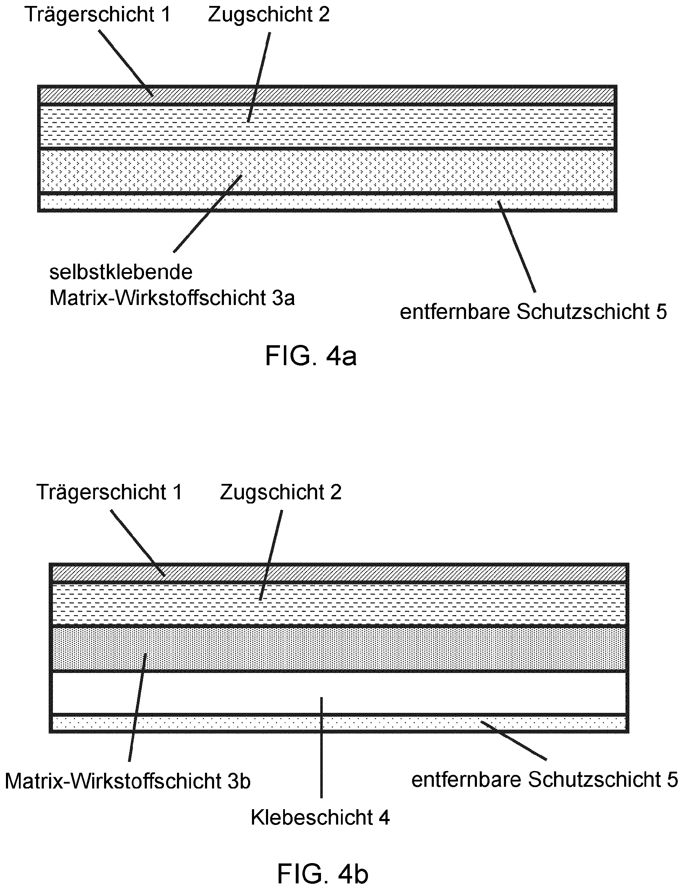

FIG. 4a zeigt eine erste Ausführungsform des externen Nasendilatators 10, wobei die Wirkstoffschicht 3 als selbstklebende Matrix-Wirkstoffschicht 3a ausgebildet ist. Ist die Wirkstoffschicht 3 als selbstklebende Matrix-Wirkstoffschicht 3a ausgebildet, so umfasst der externe Nasendilatator 10 keine Klebeschicht 4. Vielmehr bildet die selbstklebende Matrix-Wirkstoffschicht 3a in dieser Ausführungsform sowohl die Wirkstoffschicht 3 als auch die Klebeschicht 4. Bei der selbstklebenden Matrix-Wirkstoffschicht 3a ist der Wirkstoff gleichmäßig in der Schicht eingelagert und durch die direkte Haftung auf der Nasenhaut 25 erfolgt eine gleichmäßige und kontrollierte transdermale Abgabe an die Nasenhaut 25. Im Falle einer selbstklebenden Matrix-Wirkstoffschicht 3a dient die Matrix sowohl als Wirkstoffspeicher, als auch zur Fixierung des externen Nasendilatators auf der Nase 15. Die selbstklebende Matrix-Wirkstoffschicht 3a kann beispielsweise eine viskoelastische Schicht sein, in der der Wirkstoff eingebettet ist. Die selbstklebende Matrix-Wirkstoffschicht 3a passt sich also beim Andrücken and die Nase 15 gut an die Struktur der Nasenhaut 25 an, bleibt dabei aber formstabil, sodass die Zugkräfte der Zugschicht 2 ausgeübt werden können.

FIG. 4b zeigt eine zweite Ausführungsform des externen Nasendilatators 10, wobei die Wirkstoffschicht 3 als Matrix-Wirkstoffschicht 3b ausgebildet ist. Ist die Wirkstoffschicht 3 als Matrix-Wirkstoffschicht 3b ausgebildet, so umfasst der externe Nasendilatator 10 eine unter der Wirkstoffschicht 3 liegende Klebeschicht 4 zum Fixieren des externen Nasendilatators 10 auf der Nase 15. Die Matrix-Wirkstoffschicht 3b dient als Wirkstoffträger, in der der Wirkstoff gleichmäßig verteilt ist. Nach Applikation des externen Nasendilatators 10 auf der Nasenhaut 25 wird der Wirkstoff über ein Konzentrationsgefälle freigesetzt und diffundiert durch die Nasenhaut 25 in den Blutkreislauf. Die Matrix-Wirkstoffschicht 3b gibt den Wirkstoff nach Applikation auf der Nase 15 so lange kontinuierlich ab, wie ein ausreichendes Konzentrationsgefälle zwischen dem Wirkstoff in der Matrix-Wirkstoffschicht 3b und der Nasenhaut 25 vorliegt.

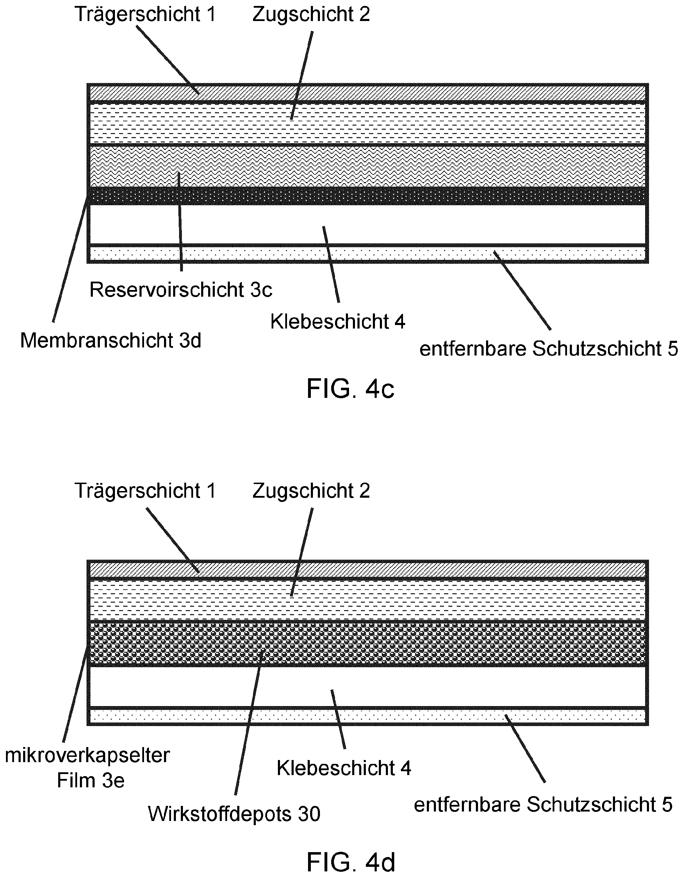

FIG. 4c zeigt eine dritte Ausführungsform des externen Nasendilatators 10, wobei die Wirkstoffschicht 3 als Reservoirschicht 3c ausgebildet ist. Ist die Wirkstoffschicht 3 als Reservoirschicht 3c ausgebildet, ist unter der Reservoirschicht 3c zusätzlich eine Membranschicht 3d angeordnet. Der Wirkstoff ist in der Reservoirschicht 3c in gelöster oder dispergierter Form enthalten. Die Membranschicht 3d dient als kontrollierende Barriere zwischen der Reservoirschicht 3c und der Nasenhaut 25, durch die der Wirkstoff mit einer bestimmten Rate abgegeben wird. Die Abgabe erfolgt über Diffusion durch die Nasenhaut 25 über ein Konzentrationsgefälle zwischen dem Wirkstoff in der Reservoirschicht 3c und der Nasenhaut 25, wobei die Membranschicht 3d sicherstellt, dass die Abgabegeschwindigkeit unabhängig von der verbleibenden Wirkstoffmenge in der Reservoirschicht 3c konstant bleibt, wodurch eine gleichbleibende Dosierung des Wirkstoffs über den Zeitraum der Applikation gewährleistet wird.

FIG. 4d zeigt eine vierte Ausführungsform des externen Nasendilatators 10, wobei die Wirkstoffschicht 3 als mikroverkapselter Film 3e ausgebildet ist. Ist die Wirkstoffschicht 3 als mikroverkapselter Film 3e ausgebildet, umfasst der mikroverkapselte Film 3e eine Mehrzahl von Wirkstoffdepots 30. Jedes Wirkstoffdepot der Mehrzahl von Wirkstoffdepots 30 enthält den Wirkstoff in verkapselter Form, sodass eine Abgabe des Wirkstoffs an die Nasenhaut 25 kontrolliert verzögert wird. Die Mehrzahl der Wirkstoffdepots 30 ist gleichmäßig über die Wirkstoffschicht 3 verteilt, sodass eine definierte gleichmäßige Abgabe über die gesamte Applikationsfläche ermöglicht wird.

Sofern nicht anders angegeben, schließen sich die vorgenannten alternativen Beispiele nicht gegenseitig aus, sondern können in verschiedenen Kombinationen eingesetzt werden, um einzigartige Vorteile zu erzielen. In der vorstehenden Beschreibung sind die beschriebenen Beispiele sowie die Formulierungen "wie", "einschließlich" und dergleichen nicht als Beschränkung der Ausführungsformen auf die spezifischen Beispiele zu verstehen, vielmehr sollen die Beispiele nur eine von vielen möglichen Ausführungsformen veranschaulichen.

## Patentansprüche

1. Externer Nasendilatator (10), umfassend:
eine Zugschicht (2), konfiguriert zum Ausüben einer Zugkraft auf Nasenflügel (20a), sodass die Nasenflügel (20a) zu geweiteten Nasenflügeln (20b) geweitet werden, wenn der externe Nasendilatator (10) auf einer Nase (15) getragen wird,
eine Wirkstoffschicht (3), konfiguriert zum transdermalen Abgeben eines Wirkstoffs an eine Nasenhaut (25),
wobei die Zugschicht (2) oberhalb der Wirkstoffschicht (3) angeordnet ist.

2. Externer Nasendilatator (10) nach Anspruch 1, wobei die Zugschicht (2) aus einem elastisch verformbaren Material besteht und die Zugschicht (2) derart vorgespannt ist, dass die auf die Nasenflügel (20a) ausgeübte Zugkraft die Nasenflügel (20a) quer zum Nasenrücken der Nase (15) zu geweiteten Nasenflügeln (20b) weitet.

3. Externer Nasendilatator (10) nach Anspruch 1 oder 2, wobei der Wirkstoff einer aus Nikotin, Tetrahydrocannabinol, THC, oder Cannabidiol, CBD, ist.

4. Externer Nasendilatator (10) nach einem der vorhergehenden Ansprüche,
wobei die Wirkstoffschicht (3) ferner Aromastoffe und/oder feuchtigkeitsspendende Substanzen und/oder oder sensorische Elemente enthält.

5. Externer Nasendilatator (10) nach einem der Ansprüche 1 bis 4,
wobei die Wirkstoffschicht (3) als selbstklebende Matrix-Wirkstoffschicht (3a) ausgebildet ist, konfiguriert zum Fixieren des externen Nasendilatators (10) auf der Nasenhaut (25) und zum transdermalen Abgeben des Wirkstoffs an die Nasenhaut (25) über ein Matrixsystem.

6. Externer Nasendilatator (10) nach einem der Ansprüche 1 bis 4,
wobei die Wirkstoffschicht (3) als Matrix-Wirkstoffschicht (3b) ausgebildet ist, konfiguriert zum transdermalen Abgeben des Wirkstoffs an die Nasenhaut (25) über ein Matrixsystem.

7. Externer Nasendilatator (10) nach Anspruch 1 bis 4,
wobei die Wirkstoffschicht (3) eine Reservoirschicht (3c) und eine Membranschicht (3d) umfasst,
wobei die Membranschicht (3d) unterhalb der Reservoirschicht (3c) angeordnet ist, und
wobei die Reservoirschicht (3c), zum kontrollierten transdermalen Abgeben des Wirkstoffs an die Nasenhaut (25) über die Membranschicht (3d) konfiguriert ist.

8. Externer Nasendilatator (10) nach Anspruch 1 bis 4,
wobei die Wirkstoffschicht (3) als mikroverkapselter Film (3e) ausgebildet ist,
wobei der mikroverkapselte Film (3e) eine Mehrzahl verkapselter Wirkstoffdepots (30) umfasst, und
wobei jedes der Mehrzahl verkapselter Wirkstoffdepots (30) zum kontrollierten transdermalen Abgeben des Wirkstoffs an die Nasenhaut (25) konfiguriert ist.

9. Externer Nasendilatator (10) nach einem der Ansprüche 6 bis 8 ferner, umfassend:
eine Klebeschicht (4), konfiguriert zum Fixieren des externen Nasendilatators (10) auf der Nasenhaut (25),
wobei die Klebeschicht (4) unterhalb der Wirkstoffschicht (3) angeordnet ist.

10. Externer Nasendilatator (10) nach einem der vorhergehenden Ansprüche, ferner umfassend:
eine Trägerschicht (1), die zum Schützen vor äußeren Einwirkungen konfiguriert ist, und
wobei die Trägerschicht (1) oberhalb der der Zugschicht (2) angeordnet ist.

11. Externer Nasendilatator (10) nach einem der vorhergehenden Ansprüche, ferner umfassend:
eine entfernbare Schutzschicht (5), die zum Schützen vor äußeren Einwirkungen vor Applikation des externen Nasendilatators (10) auf der Nasenhaut (25) konfiguriert ist,
wobei die entfernbare Schutzschicht (1) als unterste Schicht des externen Nasendilatators (10) angeordnet ist.
